# EUROPÄISCHE PATENTANMELDUNG

(11) **EP 4 015 643 A1**
(43) Veröffentlichungstag der Anmeldung: **22.06.2022**
(21) Anmeldenummer: 20214921.7
(22) Anmeldetag: 17.12.2020
(51) Int. Cl.: C12P 23/00, C12N 1/14

(54) **VERFAHREN ZUR GEWINNUNG EINES STOFFWECHSELPRODUKTES AUS PILZZELLEN**

(71) Anmelder: Helmholtz-Zentrum für Umweltforschung GmbH-UFZ, 04318 Leipzig (DE)
(72) Erfinder: Papert, Thomas, 04703 Leisnig (DE); Aurich, Andreas, 04207 Leipzig (DE); Müller, Roland Arno, 04420 Markranstädt (DE)
(74) Vertreter: Beyer, Andreas

(57) **Zusammenfassung**

Verfahren zur Gewinnung eines Stoffwechselproduktes aus Pilzzellen, aufweisend
a) Kultivieren von Pilzzellen, welche zur Erzeugung des Stoffwechselproduktes befähigt sind, in einem flüssigen wässrigen Kulturmedium, welches eine Nährstoffverbindung enthält, über einen ersten Zeitraum, wobei am Ende des ersten Zeitraums die Nährstoffverbindung verbraucht ist,
b) Belassen der Pilzzellen, nachdem die Nährstoffverbindungverbraucht ist, in dem Kulturmedium, für einen zweiten Zeitraum, in dem die Pilzzellen das Stoffwechselprodukt bilden,
wobei entweder in a) oder in b) eine lipophile Flüssigkeit vorhanden ist oder zugegeben wird, sodass eine lipophile Phase zusätzlich zu dem wässrigen Kulturmedium vorhanden ist oder gebildet wird,
c) Zugeben der Nährstoffverbindung in das wässrige Kulturmedium, und Belassen der Pilzzellen in dem Kulturmedium für einen dritten Zeitraum, in dem das Stoffwechselprodukt aus den Pilzzellen in die lipophile Phase abgegeben wird, wobei am Ende des dritten Zeitraums die Nährstoffverbindung verbraucht ist,
d) Isolieren des Stoffwechselproduktes.

## Beschreibung

### GEBIET DER ERFINDUNG

Die Erfindung betrifft ein Verfahren zur Gewinnung eines Stoffwechselproduktes aus Pilzzellen, welches in Submerskultur stattfindet.

### TECHNISCHER HINTERGRUND DER ERFINDUNG

Die Herstellung und Gewinnung von wertvollen Substanzen aus Pilzen, insbesondere Hefen, ist aus dem Stand der Technik bekannt, zum Beispiel aus Frengova, G. I.; Beshkova, D. M. Carotenoids from Rhodotorula and Phaffia: Yeasts of Biotechnological Importance. J. Ind. Microbiol. Biotechnol. 2009, 36 (2), 163;
Moline, M.; Libkind, D.; van Broock, M. Production of Torularhodin, Torulene, and β-Carotene by Rhodotorula Yeasts. Microb. Carotenoids Fungi Methods Protoc. 2012, 275-283; oder
Andrewes, A. G.; Phaff, H. J.; Starr, M. P. Carotenoids of Phaffia Rhodozyma, a Red-Pigmented Fermenting Yeast. Phytochemistry 1976, 15 (6), 1003-1007.

Jedoch sind die Wirtschaftlichkeit der Verfahren und Produktausbeuten bisher nicht befriedigend.

### AUFGABE DER ERFINDUNG

Die Aufgabe der vorliegenden Erfindung besteht darin, ein effektives Verfahren zur Herstellung von wertvollen Stoffwechselprodukten, zum Beispiel Pigmenten und Wirkstoffen aus Pilzen bereitzustellen, das hohe Produktausbeuten und die eine In-situ-Produktisolierung erlaubt.

### ZUSAMMENFASSUNG DER ERFINDUNG

Diese Aufgabe wird mit einem Verfahren gemäß Anspruch 1 gelöst. Die Unteransprüche geben bevorzugte Ausführungsformen an. Weitere Ausführungsformen sind in der Beschreibung angegeben.

Angegeben wird von der Erfindung ein Verfahren zur Gewinnung eines Stoffwechselproduktes aus Pilzzellen, aufweisend
a) Kultivieren von Pilzzellen, welche zur Erzeugung des Stoffwechselproduktes, insbesondere eines Pigmentes oder Wirkstoffes, befähigt sind, in einem flüssigen wässrigen Kulturmedium, welches eine Nährstoffverbindung enthält, über einen ersten Zeitraum, wobei am Ende des ersten Zeitraums die Nährstoffverbindung verbraucht ist,
b) Belassen der Pilzzellen, nachdem die Nährstoffverbindung verbraucht ist, in dem Kulturmedium, für einen zweiten Zeitraum, in dem die Pilzzellen das Stoffwechselprodukt bilden,
   wobei entweder eine lipohile Flüssigkeit zusätzlich zu dem wässrigen Kulturmedium vorhanden ist oder in a) oder in b) zugegeben wird, sodass eine lipophile Phase zusätzlich zu dem wässrigen Kulturmedium vorhanden ist oder gebildet wird,
c) Zugeben der Nährstoffverbindung, oder einer anderen Nährstoffverbindung für die Pilzzellen, in das wässrige Kulturmedium, und Belassen der Pilzzellen in dem Kulturmedium für einen dritten Zeitraum, in dem das Stoffwechselprodukt aus den Pilzzellen in die lipophile Phase abgegeben wird, wobei am Ende des dritten Zeitraums die Nährstoffverbindung, oder die andere Nährstoffverbindung, verbraucht ist,
d) Isolieren des Stoffwechselproduktes.

Angegeben wird von der Erfindung hiermit ein Verfahren zur biotechnologischen Herstellung von wertvollen oder brauchbaren Stoffwechselprodukten, insbesondere Pigmenten oder Wirkstoffen. Wirkstoffe sind vorzugsweise pharmakologisch aktive Wirkstoffe.

Das Stoffwechselprodukt ist ein von den den Pilzzellen hergestelltes Stoffwechselprodukt, also ein vom Stoffwechsel der Pilzzellen hervorgebrachtes Produkt.

Es kann sich bei dem Stoffwechselprodukt um ein Stoffwechselprodukt handeln, das von Wildtyp-Pilzzellen hergestellt wird.

Es kann sich bei dem Stoffwechselprodukt um ein Stoffwechselprodukt handeln, das von genetisch modifizierten, insbesondere gentechnisch modifzierten, Pilzzellen hergestellt wird, und dann vorzugsweise ein Stoffwechselprodukt ist, das nicht von Wildtyp-Pilzzellen hergestellt wird.

Die Pilze bzw. deren Zellen können insbesondere Hefen, aber auch andere Pilze z.B. filamentöse sein.

Mit dem Verfahren kann das Stoffwechselprodukt in lipophiler Phase erhalten werden, und aus dieser isoliert werden. Es ist ferner möglich, dass das Stoffwechselprodukt von der lipophilen Phase ganz oder teilweise in das wässrige Kulturmedium übergeht, wie anhand spezieller Ausführungsformen noch beschrieben, und aus dem wässrigen Kulturmedium isoliert werden kann.

Das wässrige Kulturmedium bildet in dem Verfahren eine hydrophile Phase, speziell eine wässrige Phase aus. Diese liegt neben der lipophilen Phase vor. Die wässrige Phase und die lipophile Phase sind bei Raumtemperatur, speziell im Bereich 20-25°C, flüssig. Das wässrige Kulturmedium und die lipophile Phase sind schwer oder vorzugsweise nicht miteinander mischbar.

Bei dem Verfahren erfolgt eine submerse Kultivierung der Pilze, vorzugsweise unter aeroben Bedingungen.

### AUSFÜHRLICHE BESCHREIBUNG DER ERFINDUNG

Der Ablauf des Verfahrens wird wie folgt erläutert:

In a), auch bezeichnet als Verfahrensabschnitt a) oder Abschnitt a), speziell als Schritt a), erfolgt eine Kultivierung der Pilzzellen. Hierbei erfolgt insbesondere ein Wachstum und/oder eine Vermehrung der Pilzzellen.
Am Ende des ersten Zeitraums, bzw. am Ende des Abschnitts bzw. Verfahrensabschnitts a) des Verfahrens ist die Nährstoffverbindung verbraucht oder im Wesentlichen verbraucht. Dies wird auch als Nährstofflimitation bezeichnet.

Es erfolgt dann die Produktsynthese, also die Synthese des Stoffwechselproduktes während des zweiten Zeitraums, in Abschnitt bzw. Verfahrensabschnitt b) oder im Speziellen Schritt b) des Verfahrens. Diese Synthese ist durch die intrazelluläre Akkumulierung des Stoffwechselproduktes gekennzeichnet. Die Dauer des zweiten Zeitraums ist vorzugsweise 6 bis 72 Stunden, mehr bevorzugt 10 bis 60 Stunden, besonders bevorzugt nach 6 bis 48 Stunden, meist bevorzugt 12 bis 48 Stunden.

Der Begriff, dass die Nährstoffverbindung, oder eine andere Nährstoffverbindung, verbraucht ist bedeutet vorzugsweise, dass die Nährstoffverbindung, oder die andere Nährstoffverbindung, soweit verbraucht ist, dass sich ein weiteres Wachstum und/oder weitere Vermehrung der Pilzzellen zumindest verlangsamt oder vorzugsweise zum Stillstand oder im Wesentlichen zum Stillstand kommt.

Nach der Produktbildung erfolgt vorzugsweise eine Verringerung des Sauerstoffbedarfes, welches u.a. auf die inhibierende Wirkung des eigenen Stoffwechselproduktes zurückzuführen ist.

In c), auch bezeichnet als Abschnitt c) bzw. Verfahrensabschnitt c), oder speziell Schritt c), wird die Produktsynthese durch die Zugabe der Nährstoffverbindung, oder einer anderen Nährstoffverbindung, beendet. Dies wird auch bezeichnet als Aufhebung der Nährstofflimitation. Dies geschieht vorzugsweise nach 6 bis 72 Stunden, mehr bevorzugt nach 10 bis 60 Stunden, besonders bevorzugt nach 6 bis 48 Stunden, meist bevorzugt 12 bis 48 Stunden nach Beginn der Produktsynthese. Anders ausgedrückt hat der zweite Zeitraum im Abschnitt der Produktsynthese (Abschnitt b) des Verfahrens) vorzugsweise eine solche Dauer.

In c) wird das Stoffwechselprodukt vom Organismus, der Pilzzelle, an die lipophile Phase abgegeben. Dies wird auch bezeichnet als In-situ-Extraktion des Stoffwechselproduktes.

Die lipophile Phase bzw. die lipophile Flüssigkeit dient somit als Extraktionsmittel. Hierbei kann eine kurze Wachstumsphase und/oder Vermehrungsphase des verwendeten Organismus stattfinden. Darin scheiden die Pilzzellen Stoffwechselprodukt aus. Dieses ist für die Pilzzellen überschüssiges Stoffwechselprodukt. Das Stoffwechselprodukt ist vorzugsweise lipophil und geht in die lipophile Phase. Die Dauer des dritten Zeitraums in Abschnitt c) ist vorzugsweise 6 bis 72 Stunden, oder 12 bis 72 Stunden, mehr bevorzugt 10 bis 60 Stunden, besonders bevorzugt nach 6 bis 48 Stunden, meist bevorzugt 12 bis 48 Stunden.

Das wässrige Kulturmedium (auch: Kultivierungsmedium) kann ein übliches, synthetisches, halbsynthetisches oder komplexes Nährmedium zur Kultivierung von Pilzzellen sein.

Die lipophile Flüssigkeit, welche die lipophile Phase bildet, kann als Substrat für die Pilzzellen zur Bildung des Stoffwechselproduktes dienen und wird dann im Laufe des Verfahrens in ihrer Menge verringert. Die lipophile Flüssigkeit kann somit sowohl als Extraktionsmittel für das Produkt als auch als Substrat für die Bildung des Produktes dienen.

Die Zugabe der lipophilen Flüssigkeit kann vorzugsweise gleich zu Beginn des Verfahrens, im Verlauf des Abschnitts a), oder am Ende des Abschnitts a)/zu Beginn des Abschnitts b) (Eintritt einer Nährstofflimitation), erfolgen, oder im Verlauf des Abschnitts b). Insbesondere bevorzugt kann die Zugabe 12 bis 72 h nach Eintritt einer Nährstofflimitation erfolgen, also einen solchen Zeitraum nach Beginn des Abschnitts b). Zunächst können in Abschnitt b) also die Pilzzellen in dem Kulturmedium für 12 bis 72 h belassen werden und nach diesem Zeitraum erfolgt die Zugabe der lipophilen Flüssigkeit.

In einer Ausführungsform weist das Verfahren auf: ein einfaches oder mehrfaches Wiederholen einer Sequenz von b) und c), vor der Durchführung von d). die Sequenz kann also lauten: a) - [b) - c)]ₙ - d), wobei eine ganze Zahl größer ≥1 ist. Bei n = 1 erfolgt also eine einfache Wiederholung.

Eine Wiederholung von b) bedeutet eine erneute Nährstofflimitation. Die Dauer eines erneuten Abschnitts b), ein erneuter zweiter Zeitraum, kann sein wie oben oder an anderer Stelle für den zweiten Zeitraum angegeben. Nach Beendigung des erneuten Abschnitts b) kann erneut ein Abschnitt c) erfolgen. Die Dauer eines erneuten Abschnitts c), ein erneuter dritter Zeitraum, kann sein wie oben oder an anderer Stelle für den dritten Zeitraum angegeben.
Jede weitere Substratzugabe erfolgt bevorzugt alle 6 bis 72 Stunden, mehr bevorzugt alle 10 bis 60 Stunden, besonders bevorzugt alle 6 bis 48 Stunden, meist alle 12 bis 48 Stunden nach Eintritt einer Nährstofflimitation, wobei hiermit die Dauer des erneuten Abschnitts b) bezeichnet ist.

Im Falle einer Abgabe des Stoffwechselproduktes an das Kulturmedium und eines gleichzeitigen Verbrauches der lipophilen Phase nimmt die Konzentration des Stoffwechselproduktes in der lipophilen Phase zu.

In einer Ausführungsform des Verfahrens erfolgt das Wiederholen der Sequenz von b) und c) so oft, bis das Stoffwechselprodukt aus der lipophilen Phase ausfällt.

Vorzugsweise erfolgen hierzu 2-20 Wiederholungen, auch bezeichnet als Zyklen (n, wie oben bezeichnet, gleich 2-20). Die lipophile Phase ist nach 2 bis 20 Zyklen gesättigt, sodass durch den weiteren Verbrauch der lipophilen Flüssigkeit das Stoffwechselprodukt in fester Form (Kristallform) in der lipophilen Phase ausfällt. In einer Ausführungsform des Verfahrens wird hierbei das Stoffwechselprodukt in der lipophilen Phase kristallisiert, fällt also in kristalliner Form aus.

Eine Nährstoffverbindung bezeichnet eine für die Lebenserhaltung, vorzugsweise für das Wachstum und/oder die Vermehrung, der Pilzzellen erforderlichen Stoff.

Die Nährstoffverbindung kann eine von mehreren möglichen, oder erforderlichen, Nährstoffverbindungen für die Pilzzellen sein. D.h. die Nährstoffverbindung ist möglicherweise nicht die einzige mögliche oder erforderlichen Nährstoffverbindung für die Pilzzellen. Es erfindungsgemäß möglich, das Verfahren mit mehreren Nährstoffverbindungen durchzuführen.

Die Nährstoffverbindung kann eine organische oder eine anorganische Verbindung sein.

Die Nährstoffverbindung kann eine kovalente oder eine ionische Verbindung sein.

Die Nährstoffverbindung ist vorzugsweise ausgewählt aus einer Stickstoff-haltigen Verbindung, einer Phosphor-haltigen Verbindung, einer Alkalimetall-haltigen Verbindung, einer Erdalkalimetall-haltigen Verbindung, einer Schwefel-haltigen Verbindung.

Die Stickstoff-haltige Verbindung ist vorzugsweise ausgewählt aus Harnstoff, einer Nitritverbindung, einer Nitratverbindung oder einer Ammoniumverbindung.

Die Phosphor-haltige Verbindung ist vorzugsweise ausgewählt aus einer Phosphatverbindung, einer Hydrogenphosphatverbindung, oder einer Dihydrogenphosphatverbindung.

Die Alkalimetall-haltige Verbindung ist vorzugsweise ausgewählt aus einer Kaliumverbindung.

Die Erdalkalimetall-haltige Verbindung ist vorzugsweise ausgewählt aus einer Calciumverbindung.

Die Schwefel-Verbindung ist vorzugsweise ausgewählt aus einer Sulfatverbindung.

Die "andere Nährstoffverbindung" ist eine von der Nährstoffverbindung in a) und b) (auch bezeichnet als "erste Nährstoffverbindung") des Verfahrens chemisch unterscheidbare Verbindung. Es kann sich aber beispielsweise ebenfalls um eine Stickstoff-haltige Verbindung handeln, wenn die erste Nährstoffverbindung bereits eine Stickstoff-haltige Verbindung ist (entsprechend bei einer Phosphor-haltigen Verbindung, einer Alkalimetall-haltigen Verbindung, einer Erdalkalimetall-haltigen Verbindung, oder einer Schwefel-haltigen Verbindung).

Die Nährstoffverbindung kann ein Vitamin sein, vorzugsweise Thiaminhydrochlorid.

Die lipophile Phase hat vorzugsweise einen Volumenanteil von 0,1 Volumen%, bezogen auf das Gesamtvolumen von wässrigem Kulturmedium und lipophiler Phase.

Als Kohlenstoffquelle kann zumindest eine Kohlenstoffverbindung, die in der lipophilen Phase vorhanden ist, dienen. Die zumindest eine Kohlenstoffverbindung ist dann vorzugsweise lipophil. Im speziellen kann eine Kohlenstoffquelle eine Kohlenstoffverbindung sein, welche die lipohile Flüssigkeit bildet. Der Anteil der Kohlenstoffverbindung in der lipophilen Phase beträgt vorzugsweise zumindest 0,5 Gewichtsprozent. Beispielhafte Kohlenstoffverbindungen Monocarbonsäuren, vorzugsweise Fettsäuren, wie zum gesättigte Fettsäuren oder einfach oder mehrfach ungesättigte Fettsäuren. Die lipophile Flüssigkeit bzw. die daraus gebildete lipophile Phase kann ein Öl sein, welches genannte Säuren als Bestandteil enthält, in Form von Trigylceriden zum Beispiel.

In einer Ausführungsform des Verfahrens ist in dem wässrigen Kulturmedium zumindest eine Kohlenstoffverbindung, insbesondere zumindest ein Kohlehydrat, enthalten. Diese zumindest eine Kohlenstoffverbindung ist vorzugsweise hydrophil. Diese Kohlenstoffverbindung kann zusätzlich zu einer Kohlenstoffverbindung, die in der lipophilen Phase vorhanden ist, vorhanden sein und als Kohlenstoffquelle für die Pilzzellen dienen. In diesem Fall spricht man auch von einer Mischsubstrat-Prozessführung. Ist die Kohlenstoffverbindung nur in der lipophilen Phase vorhanden, spricht man von einer Monosubstrat Prozessführung. Kohlenstoffverbindungen in dem wässrigen Kulturmedium sind vorzugsweise ausgewählt aus Hexosen, Pentosen, Stärke, Melasse, Bierwürze oder Nebenprodukten der Zucker- oder Biodieselindustrie, insbesondere Rohglycerin, Glycerinwasser oder Glycerin. Der Anteil der Kohlenstoffverbindung in dem wässrigen Kulturmedium, welches eine hydrophilen Phase ausbildet, beträgt vorzugsweise zumindest 0,1 Gewichtsprozent.

In einer Ausführungsform des Verfahrens wird in dem zweiten Zeitraum in b) die Temperatur gegenüber dem ersten Zeitraum in a) und/oder dem dritten Zeitraum in c) um 5 bis 13°C gesenkt.

In einer Ausführungsform des Verfahrens wird in dem zweiten Zeitraum in b) der pH Wert in dem wässrigen Kulturmedium gegenüber dem ersten Zeitraum in a) und/oder dem dritten Zeitraum in c) auf pH 5 abgesenkt.
Eine solche Regulierung des pH-Wertes ist vorteilhaft für das Wachstum und/oder Vermehrung und die Produktbildung. Die pH-Wert Einstellung erfolgt bevorzugt mit Substanzen wie CaCO₃, KOH, NaOH, NaHCO₃, H₂SO₄, oder HCl.

In einer Ausführungsform des Verfahrens wird in dem zweiten Zeitraum in b) gegenüber dem ersten Zeitraum in a) und/oder dem dritten Zeitraum in c) ein Überdruck von 50 Pa oder höher über dem Kulturmedium erzeugt.

In einer Ausführungsform des Verfahrens wird während des Verfahrens in a), b) und/oder c), vorzugsweise zumindest in b), die lipophile Phase durch Stoffwechselprozesse in den Pilzzellen verringert. Dies ist besonders dann der Fall, wenn eine Substanz, welche die lipophile Phase bildet oder mit bildet, als Substrat für die Pilzzellen bzw. deren Stoffwechselprozesse dient.

In einer speziellen Ausführungsform des Verfahrens wird die lipophile Phase soweit verbraucht, vorzugsweise vollständig verbraucht, dass das Stoffwechselprodukt zumindest teilweise aus der lipophilen Phase in das wässrige Kulturmedium übergeht. Dies kann zum Beispiel dadurch geschehen, dass die lipophile Phase mit dem Stoffwechselprodukt übersättigt wird. Vorteilhafterweise erfolgt hierzu, wie oben beschrieben, ein einfaches oder mehrfaches Wiederholen einer Sequenz von b) und c).

In einer speziellen Variante wird die lipophile Phase vollständig verbraucht und das Stoffwechselprodukt geht in das wässrige Kulturmedium über.

In einer Ausführungsform des Verfahrens ist das Stoffwechselprodukt ausgewählt aus einem Pigment, einem Carotinoid, einem Antibiotikum, einem Toxin, einem Statin, einem Riboflavin, einem Chlorflavin, Chaetocin, Cinnabarinsäure, einem Xanthon, einem Flutamid, oder einem Anthrachinon.

In einer Ausführungsform des Verfahrens sind die Pilzzellen ausgewählt aus,
∘ *Cryptococcus* sp.
∘ *Rhodotorula* sp.
∘ *Rhodosporidium* sp.
∘ *Yarrowia* sp.
∘ *Phaffia rhodozyma sp.*
∘ *Xanthophyllomyces dendrorhous sp.*
∘ *Neurospora crassa sp.*
∘ *Monascus sp.*
∘ *Blakeslea trispora sp.*
∘ *Mucor circinelloides sp.*
∘ *Phycomyces blakesleeanus sp.*
∘ *Penicillium oxalicum sp.*
∘ *Penicillium chrysogenum sp.*
∘ *Penicillium expansum sp.*
∘ *Fusarium sporotrichioides sp.*
∘ *Aspergillus versicolor sp.*
∘ *Pycnoporus cinnabarinus sp.*
∘ *Laetiporus sulphureus sp.*
∘ *Pseudogymnoascus roseus sp.*

Die Pilzzellen können vorzugsweise ausgewählt sein aus den folgenden Hefen:
∘ *Cryptococcus sp.*
∘ *Rhodotorula sp.*
∘ *Rhodosporidium sp.*
∘ *Yarrowia sp.*
∘ *Phaffia rhodozyma*
∘ *Xanthophyllomyces dendrorhous*

Die Pilzzellen können vorzugsweise ausgewählt sein aus den folgenden:
∘ *Neurospora crassa*
∘ *Monascus sp.*
∘ *Blakeslea trispora*
∘ *Mucor circinelloides*
∘ *Phycomyces blakesleeanus*
∘ *Penicillium oxalicum*
∘ *Penicillium chrysogenum*
∘ *Penicillium expansum*
∘ *Fusarium sporotrichioides*
∘ *Aspergillus versicolor*
∘ *Pycnoporus cinnabarinus*
∘ *Laetiporus sulphureus*
∘ *Pseudogymnoascus roseus*

Im Verfahren der Erfindung können alle bekannten und in Kultursammlungen öffentlich zugänglichen Hefen oder Pilzstämme, welche von Natur aus aber auch durch gezielte oder spontane Mutation Pigmente- oder Wirkstoffe bilden, eingesetzt werden, so z.B. *Cryptococcus curvatus* (MUCL 29819), *Rhodosporidium sphaerocarpum* (MUCL 30455), *Rhodosporidium toruloides* (MUCL 30250), *Monascus purpureus* (NBRC 4478, DSM 1379), *Pseudogymnoascus roseus* (ATCC 12262), *Monascus ruber* (DSM 1561), *Neurospora crassa* (DSM 1129), *Mucor circinelloides* (DSM 1175), *Phycomyces blakesleeanus* (DSM 1359), *Blakeslea trispora* (DSM 2387 und DSM 2388), *Aspergillus versicolor* (DSM 1943), *Fusarium sporotrichoides* (DSM 23368), *Penicillium oxalicum* (DSM 898), *Xanthophyllomyces dendrorhous, Penicillium chrysogenum* (DSM 844), *Penicillium expansum* (DSM 1282), *Yarrowia lipolytica* (DSM 1345), *Laetiporus sulphureus* (DSM 2785) und *Pycnoporus cinnabarinus* (DSM 1184).

Die Pilzzellen können Wildtyp-Pilzzellen sein. Die Wildtyp-Pilzzellen können Wildtyp-Pilzzellen von in dieser Beschreibung speziell genannten Spezies sein, wie von vorangehend genannten Spezies.

Die Pilzzellen können genetisch modifizierte Pilzzellen, insbesondere gentechnisch modifizierte Pilzzellen, sein, welche ein Stoffwechselprodukt bilden, welches natürlicherweise von den Pilzzellen, also von Wildtyp-Pilzzellen, nicht gebildet wird. Die genetisch modifizierten, insbesondere gentechnisch modifizierten, Pilzzellen können genetisch modifizierte Pilzzellen, insbesondere gentechnisch modifizierte, von in dieser Beschreibung speziell genannten Spezies sein, wie von vorangehend genannten Spezies. Unter einer gentechnisch modifizierten Pilzzelle (oder Pilzzellen) wird eine Pilzzelle verstanden, deren genetisches Material in einer Weise verändert worden ist, wie sie unter natürlichen Bedingungen durch Kreuzen oder natürliche Rekombination nicht vorkommt.

In einer Ausführungsform des Verfahrens ist die lipophile Flüssigkeit, welche die lipophile Phase ausbildet, ein pflanzlich oder tierisch produziertes Öl, also ein tierisches Öl oder ein Pflanzenöl. Beispielhaft hierfür sind Rapsöl, Maiskeimöl, Olivenöl, Sonnenblumenöl oder eine Kombination davon. In einer weiteren Variante ist die lipophile Flüssigkeit ein Abfallprodukt aus der Lebensmittelindustrie, beispielsweise Frittierfett.

In einer Ausführungsform des Verfahrens hat der erste und /oder der dritte Zeitraum eine Dauer von 6 bis 72 Stunden, vorzugsweise 12 bis 72 Stunden.

In einer Ausführungsform des Verfahrens hat der zweite Zeitraum eine Dauer von 6 bis 72 Stunden, vorzugsweise 12 bis 48 Stunden.

In einer speziellen Variante des Verfahrens wird der Pilz bis zur vollständigen morphologischen Ausbildung vorkultiviert. Daran können sich die oben beschriebenen Verfahrensabschnitte oder Schritte anschließen, beispielsweise kann der Pilz dann in einen Schüttelkolben oder Bioreaktor überführt werden, indem die weiteren Verfahrensabschnitte oder Schritte durchgeführt werden.

Eine Vorkultivierung erfolgt beispielsweise unter folgenden Bedingungen, einzeln oder in Kombination:
- bei einem pH-Wert von ≥ 2,0 bis ≤ 8,0; vorzugsweise bei einem pH-Wert von ≥3,0 bis ≤70
- einer Temperatur von ≥ 12 bis ≤ 40 °C, vorzugsweise einer Temperatur von ≥ 15 bis ≤ 35°C.

Eine Vorkultivierung kann stammspezifisch erfolgen.

Das Verfahren kann in einem Reaktor erfolgen, der auch als Bio-Reaktor bezeichnet wird.

Es ist erfindungsgemäß bevorzugt, den spezifischen Leistungseintrag in dem Verfahren, insbesondere in den Abschnitten a), b) und/oder c), vorzugsweise in Abschnitt a) und/oder b), im Reaktor auf ≥ 0,74 bis ≤ 742,8 W m⁻³, besonders bevorzugt zwischen ≥ 5,94 bis ≤ 254,78 W m⁻³, einzustellen.

Es ist erfindungsgemäß bevorzugt, dass der Begasungsvolumenstrom in dem Verfahren, insbesondere in den Abschnitten a), b) und/oder c), vorzugsweise in Abschnitt a) und/oder b), 0,5 bis 4,0 vvm (Volumen Luft/Volumen Fermenter/Minute) beträgt.

Das Verfahren kann als Repeated-Fed-Batch, Fed-Batch oder kontinuierlich betrieben werden.

Schritt oder Abschnitt d) des Verfahrens kann nachdem Fachmann allgemein bekannten Methoden durchgeführt werden, zum Beispiel durch Fest-Flüssigtrennung bzw. Extraktion vorhanden Feststoffes, gegebenenfalls nach schonender Trocknung vorhandenen Feststoffes.

Weitere Erfindungsgegenstände sind folgende:
Ein Öl oder Ölgemisch, enthaltend ein Pilzstoffwechselprodukt, dadurch gekennzeichnet, dass das Öl oder Ölgemisch nach einem vorangehend beschriebenen Verfahren hergestellt ist.

Das Öl oder Ölgemisch ist insbesondere dadurch gekennzeichnet, dass das Öl oder Ölgemisch eine Konzentration an Stoffwechselprodukt von 5 mg/L oder mehr aufweist.

Das Öl oder Ölgemisch kann eine Masse von mindestens 10 µg oder mehr des Stoffwechselproduktes, insbesondere in kristalliner Form, aufweisen.

Eine wässrige Phase enthaltend ein Pilzstoffwechselprodukt, dadurch gekennzeichnet, dass die wässrige Phase nach einem vorangehend beschriebenen Verfahren hergestellt ist.

Die wässrige Phase kann eine Masse von mindestens 10 µg oder mehr des Stoffwechselproduktes, insbesondere in kristalliner Form, aufweisen.

### BEISPIELE

Die vorliegende Erfindung wird durch die im Folgenden diskutierten Experimente genauer erläutert. Dabei ist zu beachten, dass die Figuren nur beschreibenden Charakter haben und nicht dazu gedacht sind, die Erfindung in irgendeiner Form einzuschränken. In den nachfolgenden Beispielen wird die Erfindung näher beschrieben.

### BEISPIEL 1

Der Wildtyp-Stamm *Cryptococcus curvatus* MUCL 29819 wurde jeweils auf modifizierten Setlik-Agarplatten folgender Zusammensetzung für 24 bis 48 Stunden bei 26 °C kultiviert.

| | |
|---|---|
| Kaliumdihydrogenphosphat | 0,34 g/L |
| Magnesiumsulfat-Heptahydrat | 0,99 g/L |
| Kaliumnitrat | 2,02 g/L |
| Eisensulfat-Heptahydrat | 6,40 mg/L |
| Thiaminhydrochlorid | 1,00 mg/L |
| Spurensalzlösung | 10 mL/L |
| Glucose | 20 g/L |

| | |
|---|---|
| Calciumchlorid-Dihydrat | 5,0 mg/L |
| Borsäure | 3,09 mg/L |
| Mangansulfat-Pentahydrat | 1,2 mg/L |
| Cobaltsulfat-Heptahydrat | 2,35 mg/L |
| Kupfersulfat-Pentahydrat | 1,24 mg/L |
| Zinksulfat-Heptahydrat | 1,43 mg/L |
| Ammoniummolybdat-Tetrahydrat | 1,84 mg/L |

Von den gewachsenen Agarplatten wurden jeweils 1 bis 2 Impfösen auf 100 mL sterilisiertes, mit destilliertem Wasser hergestelltes Nährmedium folgender Zusammensetzung überimpft.

| | |
|---|---|
| Kaliumdihydrogenphosphat | 0,34 g/L |
| Magnesiumsulfat-Heptahydrat | 0,99 g/L |
| Kaliumnitrat | 2,02 g/L |
| Eisensulfat-Heptahydrat | 6,40 mg/L |
| Thiaminhydrochlorid | 1,00 mg/L |
| Spurensalzlösung | 10 mL/L |
| Rapsöl | 60 g/L |

| | |
|---|---|
| Calciumchlorid-Dihydrat | 5,0 mg/L |
| Borsäure | 3,09 mg/L |
| Mangansulfat-Pentahydrat | 1,2 mg/L |
| Cobaltsulfat-Heptahydrat | 2,35 mg/L |
| Kupfersulfat-Pentahydrat | 1,24 mg/L |
| Zinksulfat-Heptahydrat | 1,43 mg/L |
| Ammoniummolybdat-Tetrahydrat | 1,84 mg/L |

Die Vorkultur wurde in 500 mL-Erlenmeyerkolben mit Schikanen, bei 26 °C, bei pH 5,0 bis 7, für 48 h auf einer Schüttelapparatur bei einer Schüttelsequenz von 150 rpm kultiviert.

Von der Vorkultur erfolgte jeweils die Überimpfung von 10 mL Inokulum auf 100 mL, sterilisiertes, mit destilliertem Wasser hergestelltes Nährmedium (Zusammensetzung siehe oben).

Die Kultivierung erfolgte unter sterilen Bedingungen bei einer Temperatur von 26 °C und einer Schüttelfrequenz von 150 rpm. Der pH-Wert wurde in einem Bereich unterhalb von 5,0 mit 15 % NaOH, sowie oberhalb von 7,0 mit HCl (2N) korrigiert.
Nach Verbrauch der Stickstoffquelle findet die Produktbildung statt, welche nach 18 h durch die Zugabe von 0,5 g/L Kaliumnitrat aufgehoben wurde. Innerhalb der folgenden 12 h fand eine Anreicherung von Carotinoiden im Rapsöl statt. Nach zwei weiteren Zyklen wurde der Versuch beendet und die carotinoidhaltige Biomasse und das carotinoidhaltige Öl untersucht. Zum Versuchsende lagen 2,88*10⁸ Zellen mL⁻¹ im Bioreaktor mit einem Carotinoidanteil in der Biomasse von 0,03 % und einer Konzentration an Carotinoiden in der Ölphase von 95,35 mg L⁻¹ vor.

### BEISPIEL 2

Der filamentösen Pilz *Blakeslea trispora* (DSM 2388) wurden zunächst auf YPsS-Festmedium (Stärke: 15 g/L, Hefeextrakt: 4 g/L, Magnesiumsulfat: 0,45 g/L, Dikaliumhydrogenphosphat: 1,0 g /L, Agar: 20 g/L) für 72 h kultiviert. Nach 3 Tagen können die Sporen mit einer 0,2 % Span20 Lösung steril abgespült werden.

Ein 300 mL Kolben wird mit 100 mL Malzmedium (Malzextrakt: 50 g/L, Sonnenblumenöl 60 g/L) befüllt. Das bei 121 °C für 20 Minuten dampfsterilisierte Medium wird nach Abkühlen auf Raumtemperatur mit jeweils 1 bis 3*10⁶ Sporen beimpft. Der Pilz wird bei 28 °C bei einer Schüttelfrequenz von 145 rpm kultiviert. Es wird keine Regulierung des pH-Wertes vorgenommen.

Nach Verbrauch der Stickstoffquelle findet die Produktbildung statt, welche nach 36 h durch die Zugabe von 1,0 g/L Malzextrakt aufgehoben wurde. Innerhalb der folgenden 12 h fand eine Anreicherung von Carotinoiden im Sonnenblumenöl statt. Nach vier weiteren Zyklen wurde der Versuch beendet und die carotinoidhaltige Biomasse und das carotinoidhaltige Öl untersucht. Zum Versuchsende lag eine Biomasse von 32,67 g/L im Schüttelkolben mit einem Carotinoidanteil von 2,28 % und einer Konzentration an Carotinoiden in der Ölphase von 473,2 mg/L vor.

### BEISPIEL 3

Mit dem Hefestamm *Rhodosporidium sphaerocarpum* (MUCL 30455) erfolgte die Kultivierung wie im Beispiel 1 beschrieben. Nach 229 h lag eine Zellzahl von 1,56*10⁹ Zellen/mL im Bioreaktor mit einem Carotinoid-Anteil von 0,04 % und einer Konzentration an Carotinoiden in der Ölphase von 105,43 mg/L vor.

### BEISPIEL 4

Der Pilz *Monascus purpureus* wurde zunächst auf YpsS-Festmedium (Stärke: 15 g/L, Hefeextrakt: 4 g/L, Magnesiumsulfat: 0,45 g/L, Dikaliumhydrogenphosphat: 1,0 g /L, Agar: 20 g/L) für 72 h kultiviert. Nach 3 bis 5 Tagen können die Sporen mit einer 0,2 % Span20 Lösung steril abgespült werden.

Ein 300 mL Kolben wird mit 100 mL Kulturmedium (stärkehaltiges Abprodukt: 20 g/L, Sonnenblumenöl 60 g/L) befüllt. Das bei 121 °C für 20 Minuten dampfsterilisierte Medium wird nach Abkühlen auf Raumtemperatur mit jeweils 1 bis 3*10⁶ Sporen beimpft. Der Pilz wird bei 28 °C bei einer Schüttelfrequenz von 145 rpm kultiviert. Es wird keine Regulierung des pH-Wertes vorgenommen.

Nach Verbrauch der Stickstoffquelle findet die Produktbildung statt, welche nach 36 h durch die Zugabe von 1,0 g/L stärkehaltiges Abprodukt aufgehoben wurde. Innerhalb der folgenden 12 h fand eine Anreicherung von Monascus-Pigmenten im Sonnenblumenöl statt. Nach vier weiteren Zyklen wurde der Versuch beendet und die farbstoffhaltige Biomasse und die kristallinen Monascus-Pigmente untersucht. Zum Versuchsende lag eine Biomasse von 23,85 g/L sowie 1,180 g/L kristalline Monascus-Pigmente im Schüttelkolben vor.

### BEISPIEL 5

Mit dem Pilz *Monascus roseus* erfolgte die Kultivierung wie im Beispiel 4 beschrieben. Nach 269 h lag eine Pilzbiomasse von 17,91 g/L sowie 0,480 g/L Pigmentkristalle im Schüttelkolben vor.

### BEISPIEL 6

Der Pilz *Pycnoporus cinnabarinus* (DSM 1184) wurden zunächst auf YPsS-Festmedium (Stärke: 15 g/L, Hefeextrakt: 4 g/L, Magnesiumsulfat: 0,45 g/L,
Dikaliumhydrogenphosphat: 1,0 g /L, Agar: 20 g/L) für 94 h kultiviert. Nach 4 Tagen können die Sporen mit einer 0,2 % Span20 Lösung steril abgespült werden.

Ein 300 mL Kolben wird mit 100 mL YPsS-Medium (Stärke: 20 g/L, Hefeextrakt: 4 g/L, Magnesiumsulfat: 0,45 g/L, Dikaliumhydrogenphosphat: 1,0 g /L, Eisensulfat: 0,64 g/L, Sonnenblumenöl 60 g/L) befüllt. Das bei 121 °C für 20 Minuten dampfsterilisierte Medium wird nach Abkühlen auf Raumtemperatur mit jeweils 1 bis 3*10⁶ Sporen beimpft. Der Pilz wird bei 28 °C bei einer Schüttelfrequenz von 120 rpm kultiviert. Es wird keine Regulierung des pH-Wertes vorgenommen.

Nach Verbrauch der Phosphatquelle findet die Produktbildung statt, welche nach 4 h durch die Zugabe von 0,1 g/L Kaliumdihydrogenphosphat aufgehoben wurde. Innerhalb der folgenden 12 h fand eine Anreicherung von Pigmenten im Sonnenblumenöl statt. Nach einem weiteren Zyklen wurde der Versuch beendet und die pigmenthaltige Biomasse und das pigmenthaltige Öl untersucht. Zum Versuchsende lag eine Biomasse von 21,03 g/L im Schüttelkolben sowie 3,6 g/L Pigmente in kristallinen Aggregatzustand vor.

### BEISPIEL 7

Mit dem Pilz *Penicillium chrysogenum* (DSM 844) erfolgte die Kultivierung wie im Beispiel 6 beschrieben. Nach 190 h lag eine Pilzbiomasse von 20,31 g/L sowie 2,48 g/L Wirkstoffkristalle im Schüttelkolben vor.

### BEISPIEL 8

Mit dem Pilz *Laetiporus sulphureus* (DSM 2785) erfolgte die Kultivierung wie im Beispiel 6 beschrieben. Nach 239 h lag eine Pilzbiomasse von 20,19 g/L sowie eine Carotinoid-Konzentration in der lipophilen Phase von 192 mg/L vor.

## Patentansprüche

1. Verfahren zur Gewinnung eines Stoffwechselproduktes aus Pilzzellen, aufweisend
a) Kultivieren von Pilzzellen, welche zur Erzeugung des Stoffwechselproduktes befähigt sind, in einem flüssigen wässrigen Kulturmedium, welches eine Nährstoffverbindung für die Pilzzellen enthält, über einen ersten Zeitraum, wobei am Ende des ersten Zeitraums die Nährstoffverbindung verbraucht ist,
b) Belassen der Pilzzellen, nachdem die Nährstoffverbindung verbraucht ist, in dem Kulturmedium für einen zweiten Zeitraum, in dem die Pilzzellen das Stoffwechselprodukt bilden,
wobei eine lipophile Flüssigkeit zusätzlich zu dem wässrigen Kulturmedium vorhanden ist oder die lipophile Flüssigkeit in a) oder in b) zugegeben wird, sodass eine lipophile Phase zusätzlich zu dem wässrigen Kulturmedium vorhanden ist oder gebildet wird,
c) Zugeben der Nährstoffverbindung, oder einer anderen Nährstoffverbindung für die Pilzzellen, in das wässrige Kulturmedium, und Belassen der Pilzzellen in dem Kulturmedium für einen dritten Zeitraum, in dem das Stoffwechselprodukt aus den Pilzzellen in die lipophile Phase abgegeben wird, wobei am Ende des dritten Zeitraums die Nährstoffverbindung, oder die andere Nährstoffverbindung, verbraucht ist,
d) Isolieren des Stoffwechselproduktes.

2. Verfahren nach Anspruch 1, aufweisend ein einfaches oder mehrfaches Wiederholen einer Sequenz von b) und c), bevor d) durchgeführt wird.

3. Verfahren nach Anspruch 2, wobei das Wiederholen so oft erfolgt, bis das Stoffwechselprodukt aus der lipophilen Phase ausfällt.

4. Verfahren nach einem der vorangehenden Ansprüche, wobei das Stoffwechselprodukt in der lipophilen Phase kristallisiert.

5. Verfahren nach einem der vorangehenden Ansprüche, wobei in dem wässrigen Kulturmedium zumindest eine Kohlenstoffverbindung enthalten ist.

6. Verfahren nach einem der vorangehenden Ansprüche, wobei in dem zweiten Zeitraum in b) die Temperatur gegenüber dem ersten Zeitraum in a) und/oder dem dritten Zeitraum in c) um 5 bis 13°C gesenkt wird.

7. Verfahren nach einem der vorangehenden Ansprüche, wobei in dem zweiten Zeitraum in b) der pH Wert in dem wässrigen Kulturmedium gegenüber dem ersten Zeitraum in a) und/oder dem dritten Zeitraum in c) auf pH 5 abgesenkt wird.

8. Verfahren nach einem der vorangehenden Ansprüche, wobei in dem zweiten Zeitraum in b) gegenüber dem ersten Zeitraum in a) und/oder dem dritten Zeitraum in c) ein Überdruck von 50 Pa oder höher über dem Kulturmedium erzeugt wird.

9. Verfahren nach einem der vorangehenden Ansprüche, wobei während des Verfahrens in a), b) und/oder c), vorzugsweise zumindest in b), die lipophile Phase durch Stoffwechselprozesse in den Pilzzellen verringert wird.

10. Verfahren nach Anspruch 9, wobei die lipophile Phase soweit verbraucht wird oder vollständig verbraucht wird, dass das Stoffwechselprodukt zumindest teilweise aus der lipophilen Phase in das wässrige Kulturmedium übergeht.

11. Verfahren nach einem der vorangehenden Ansprüche, wobei das Stoffwechselprodukt ausgewählt ist aus einem Pigment, einem Carotinoid, einem Antibiotikum, einem Toxin, einem Statin, einem Riboflavin, einem Chlorflavin, Chaetocin, Cinnabarinsäure, einem Xanthon, einem Flutamid, oder einem Anthrachinon.

12. Verfahren nach einem der vorangehenden Ansprüche, wobei die Pilzzellen ausgewählt sind aus,
∘ *Cryptococcus* sp.
∘ *Rhodotorula* sp.
∘ *Rhodosporidium* sp.
∘ *Yarrowia* sp.
∘ *Phaffia rhodozyma sp.*
∘ *Xanthophyllomyces dendrorhous sp.*
∘ *Neurospora crassa sp.*
∘ *Monascus sp.*
∘ *Blakeslea trispora sp.*
∘ *Mucor circinelloides sp.*
∘ *Phycomyces blakesleeanus sp.*
∘ *Penicillium oxalicum sp.*
∘ *Penicillium chrysogenum sp.*
∘ *Penicillium expansum sp.*
∘ *Fusarium sporotrichioides sp.*
∘ *Aspergillus versicolor sp.*
∘ *Pycnoporus cinnabarinus sp.*
∘ *Laetiporus sulphureus sp.*
∘ *Pseudogymnoascus roseus sp.*

13. Verfahren nach einem der vorangehenden Ansprüche, wobei die lipophile Flüssigkeit, welche die lipophile Phase ausbildet, ein pflanzlich oder tierisch produziertes Öl ist.

14. Verfahren nach einem der vorangehenden Ansprüche, wobei der erste und /oder der dritte Zeitraum eine Dauer von 6 bis 72 Stunden, vorzugsweise 12 bis 72 Stunden, hat.

15. Verfahren nach einem der vorangehenden Ansprüche, wobei der zweite Zeitraum eine Dauer von 6 bis 72 Stunden, vorzugsweise 12 bis 48 Stunden, hat.
